Europäisches Patentamt

**European Patent Office**

Office européen des brevets

(11) Publication number: **0 114 818**
**B1**

(12) **EUROPEAN PATENT SPECIFICATION**

(45) Date of publication of patent specification: **12.08.87**

(21) Application number: **82902927.1**

(22) Date of filing: **09.08.82**

(86) International application number:
**PCT/US82/01078**

(87) International publication number:
**WO 84/00758 01.03.84 Gazette 84/06**

(51) Int. Cl.⁴: **G 01 N 33/576,**
G 01 N 33/543

(54) **IMMUNOASSAY FOR CARBOHYDRATE ANTIGENIC DETERMINANT.**

(43) Date of publication of application:
**08.08.84 Bulletin 84/32**

(45) Publication of the grant of the patent:
**12.08.87 Bulletin 87/33**

(84) Designated Contracting States:
**AT BE CH DE FR GB LI LU NL SE**

(56) References cited:
**US-A-3 697 638**
**US-A-3 867 363**
**US-A-3 956 258**
**US-A-4 180 499**

**US,N, Fed. Proc., Vol. 41, issued 05 Mar 1982,**
**Magnani et al., see page 898, ref. no. 3672**
**US,N, Science, Vol. 212, issued 03 Apr 1981,**
**Koprowski et al., see pages 53-55**
**US,N, Science, Vol. 212, issued 03 Apr 1981,**
**Magnani et al., see pages 55-6**

(73) Proprietor: **CENTOCOR, INC.**
**244 Great Valley Parkway**
**Malvern, PA 19355 (US)**

(72) Inventor: **DelVILLANO, Jr., Bert, C.**
**529 Sugartown Road**
**Berwyn, PA 19312 (US)**
Inventor: **LIU, Yu-Sheng, V.**
**805 Charleston Greene King & Sugartown Roads**
**Malvern, PA 19355 (US)**

(74) Representative: **Holdcroft, James Gerald, Dr.**
**et al**
**Graham Watt & Co. Riverhead**
**Sevenoaks Kent TN13 2BN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 0 114 818

## Description

This invention is in the field of immunology and particularly relates to an immunoassay which can be employed by hospitals or clinical laboratories.

Monoclonal antibodies are antibodies which have been produced by a cell line cloned from a single antibody producing cell. Monoclonal antibodies are extraordinarily pure, uniform and reproducible since each antibody is effective against a single antigenic determinant.

Monoclonal antibodies can be obtained in significant quantities from hybridoma cells. Hybridoma cells are fused cells resulting from the fusion of antibody producing cells with tumor cells. The initial work relating to the production of such hybridoma cells was done by Cesar Milstein and George Kohler employing mouse myeloma cells with spleen cells taken from mice immunized with sheep red blood cells. See, Kohler et al., *Eur. J. Immunol., 6*, 511—19 (1976); Kohler et al., *Nature, 256*, 495—7 (1975); and Milstein, *Scientific American, 243 (4)*, 66—74 (1980).

More recently, Hilary Koprowski and colleagues have extended the original hybridoma work by developing hybridoma cell lines capable of producing monoclonal antibodies against specific viruses and tumors. See, U.S. Patent Nos. 4,196,265 and 4,172,124, respectively. Wands and Zurawski further extended hybridoma technology to produce hybridoma cell lines capable of producing monoclonal antibodies to hepatitis virus. See U.S. Patent No. 4,271,145. It has also been recently discovered that monoclonal IgM antibodies produced from hybridoma cell lines can be employed in certain immunoassays to improve the sensitivity and specificity thereof. See, WO 82/101072—A—published on 1st April 1982 in the name of Massachusetts General Hospital.

Even more recently, a monoclonal antibody, designated 1116 NS 19—9 (hereinafter "19—9 antibody"), was developed by Koprowski et al. by immunization of BALB/c mice with a human colorectal cancer cell line, SW1116. See Koprowski, H., Steplewski, Z., Mitchell, K., Herlyn, M., Herlyn, D., and Fuhrer, P., Colorectal carcinoma antigens detected by hybridoma antibodies, *Somatic Cell Genetics, 5*, 957—972, (1979). It has been shown that the 19—9 antibody reacts with a carbohydrate antigenic determinant (hereinafter referred to as "CA 19—9") which has been identified as a sialylated lacto-N-fucopentaose II, an oligosaccharide which shares structural features with Lewis blood group substances. See Magnani, J., Nilsson, B., Brockhaus, M., Zopf, D., Steplewski, Z., Koprowski, H., Ginsburg, V., The antigen of a tumor—specific monoclonal antibody is a ganglioside containing sialylated lacto—N—Fucopentaose II, *Fed. Proc. 41*, 898, (1982).

In early studies using a competition radio-immunoassay, the 19—9 antibody was shown to have high sensitivity in identifying patients with gastrointestinal adenocarcinomas and to have high specificity for normal individuals. See Koprowski, H., Herlyn, H., Steplewski, Z., and Sears, H. F., Specific antigen in serum of patients with colon carcinoma, *Science, 212*, 53, (1981); and, Herlyn, M., Clark, W. H., Mastrangelo, J. J., Guerry, D., Elder, D. E., Larossa, D., Hamilton, R., Bondi, E., Tutkill, R., Steplewski, Z., Koprowski, H., Specific antigens to colorectal carcinoma in sera of patients are detected by monoclonal antibodies, *Cancer Res. 40*, 3602—3609, (1982). The CA 19—9 epitope has also been identified on a glycolipid extracted from SW1116 cells and from meconium, as well as on a glycoprotein from SW1116 cells. See Magnani, J., Brockhaus, M., Smith, D., Ginsburg, V., Blaszcyk, M., Mitchell, D., Steplewski, Z., and Koprowski, H., A Monoclonal Antibody Defined Antigen of colon Carcinoma, *Science 212*, 55, (1981).

The present invention is concerned with an immunoassay for CA 19—9, and surprisingly it has been found that in such an immunoassay higher signal/noise ratios are obtainable by carrying out the antigen-antibody reaction at an acidic pH.

The resulting assay has high sensitivity for patients with gastrointestinal adenocarcinomas, especially pancreatic cancer, and has very high specificity, even among patients with benign gastrointestinal disease.

The invention provides an immunoassay for the carbohydrate antigenic determinant, CA 19—9, characterised in that the reaction between CA 19—9 antigen and CA 19—9 antibody is carried out at an acidic pH.

In one embodiment for example a forward sandwich immunoassay is employed to detect the CA 19—9 antigen. In this assay, a patient sample containing the antigen is initially incubated with a solid-phase immunoadsorbent containing immobilized 19—9 antibody. Incubation is continued for a sufficient period of time to allow antigen in the patient sample to bind to immobilized antibody on the solid-phase immunoadsorbent. After this first incubation, the solid-phase immunoadsorbent is separated from the incubation mixture and washed to remove exces antigen and other interferring substances, such as non-specific binding proteins, which also may be present in the patient sample. The solid-phase immunoadsorbent containing antigen bound to immobilized antibody is subsequently incubated for a second time with labelled antibody capable of binding to CA 19—9 antigen. After the second incubation, another wash is performed to remove unbound labelled antibody from the solid-phase immunoadsorbent thereby removing non-specifically bound labelled antibody. Labelled antibody bound to the solid-phase immunoadsorbent is then detected and the amount of labelled antibody detected serves as a direct measure of the amount of CA 19—9 antigen present in the original patient sample.

There now follows a description, to be read with reference to the accompanying drawings of experimental work illustrating the invention.

In the accompanying drawings:—

Figure 1 is a standard curve prepared for CA 19—9.

Figure 2 is a depiction of the CA 19—9 concentration in patient sera for patients having different types of cancer or benign diseases.

Figure 3 is a plot illustrating the frequency of various CA 19—9 concentrations in sera from normal blood bank donors.

Although most of the experimental work described below was carried out at a pH of 4.5, it should be recognized that other acidic pHs could have been employed. The preferred pH range was found to be from 2.5 to 6.5, and it is especially preferred to operate at a pH of about 4.5.

Similarly, the experimental work described herein relates to a forward sandwich assay. Nevertheless, it is believed that carbohydrate antigenic determinants can be determined in many cases employing acidic conditions with reverse sandwich assays or simultaneous sandwich assays.

In addition to labelling antibody with radioactive labels, other labels, such as fluorescent materials, could also be employed. Further, enzyme labels could be employed, in which case detection could be achieved by a colorimetric method employing a competing substrate for the enzyme label.

The forward sandwich assay for CA 19—9 will now be more specifically described.

Murine hybridoma 1116-NS-19—9 was obtained from Dr. Z. Steplewski (Wistar Institute, Philadelphia, PA). Hybridoma cells were grown in tissue culture and were used to prepare ascitic fluid in pristane primed BALB/c mice. IgG was purified from ascites by affinity chromatography on protein A-Sepharose® (Pharmacia) using elution at pH 4.0. Purity of the isolated IgG was demonstrated by SDS polyacrylamide gel electrophoresis, isoelectric focusing and immunoelectrophoresis.

Purified antibody was radioiodinated using $^{125}I$ labelled N-hydroxysuccinimide ester of p-hydroxy-liodophenylpropionic acid, Bolton-Hunter reagent, using the manufacturer's recommended procedure. Specific activity of the $^{125}I$ IgG ranged from 5 to 15 μCi/μgm. Polystyrene beads (1/4″; Precision Plastic Ball Co., Chicago, IL) were coated with purified antibody and dried.

Experimental samples were assayed in duplicate using a "forward sandwich" radioimmunometric assay. One hundred microliters of sera, standard solutions or controls were mixed with 100 μl of buffer (5 mg/ml bovine serum albumin, Armour Pharmaceutical, So. Plainfield, NJ; 50 mM sodium citrate; 1 mM ethylene diamine tetracetic acid; pH 3.0) in a reaction tray. An antibody coated bead was added and samples were incubated at 37° for 3 hours. Beads were washed with deionized water, then 200 μl of $^{125}I$ labelled 19—9 antibody (130,000 dpm; 5 mg/ml bovine serum albumin; 50 mM sodium citrate; pH 4.5) was added. Samples were then incubated for 3 hours at room temperature (18—22°). Beads were washed and then counted in a gamma counter.

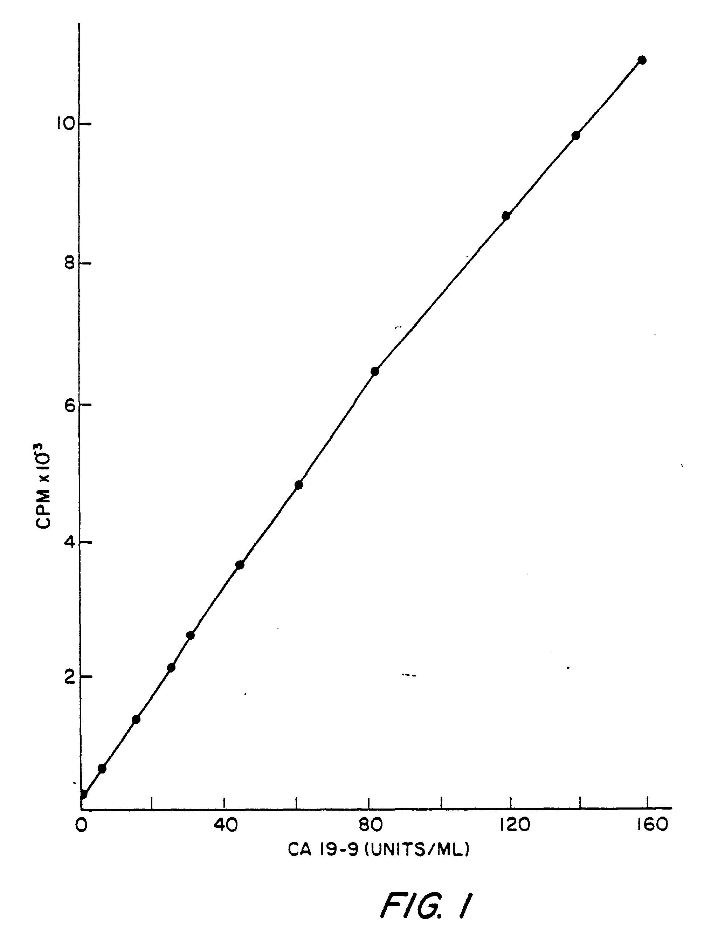

Experimental results were converted into units by comparison with a standard curve prepared as follows. Partially purified CA 19—9 was diluted into pooled reconstituted human serum to give concentrations between 6 and 160 units/ml. One hundred microliters of standards were mixed with 100 μl of buffer and an 19—9 antibody coated bead for 3 hours at 37°. Beads were washed with $dH_2O$ then $^{125}I$ labelled 19—9 antibody (130,000 dpm) was added and incubated for 3 hours at 20°C. After washing, $^{125}I$ bound to beads was determined using a gamma counter. The resulting standard curve is illustrated as Figure 1.

Frozen coded samples from the National Cancer Institute (NCI) serum bank were provided by Dr. R. Herberman (NCI). Serum was carefully prepared and frozen continuously at −70°. Clinical data were compiled from reports provided by Dr. V. Go (Mayo Clinic). Additional serum samples from random healthy blood bank donors were obtained from Dr. J. Menitove, Southeastern Blood Center of Milwaukee, Wisconsin. All donors qualified as blood donors, and were negative when tested for hepatitis B surface antigen. Data concerning age and sex were provided by Dr. Menitove.

The levels of CA 19—9 were determined by plotting [$^{125}I$]—19—9 bound versus the concentration of CA 19—9 in standard solutions. The CA 19—9 concentration of each standard was specified in arbitrary units. As shown in Figure 1, the amount of $^{125}I$ labelled 19—9 antibody bound approximated a linear function of concentration up to at least 80 units/ml. The intra- and interassay coefficients of variation were 5—12% in the range between 5 and 120 units/ml and the minimum detectable dose was 1.5 units/ml.

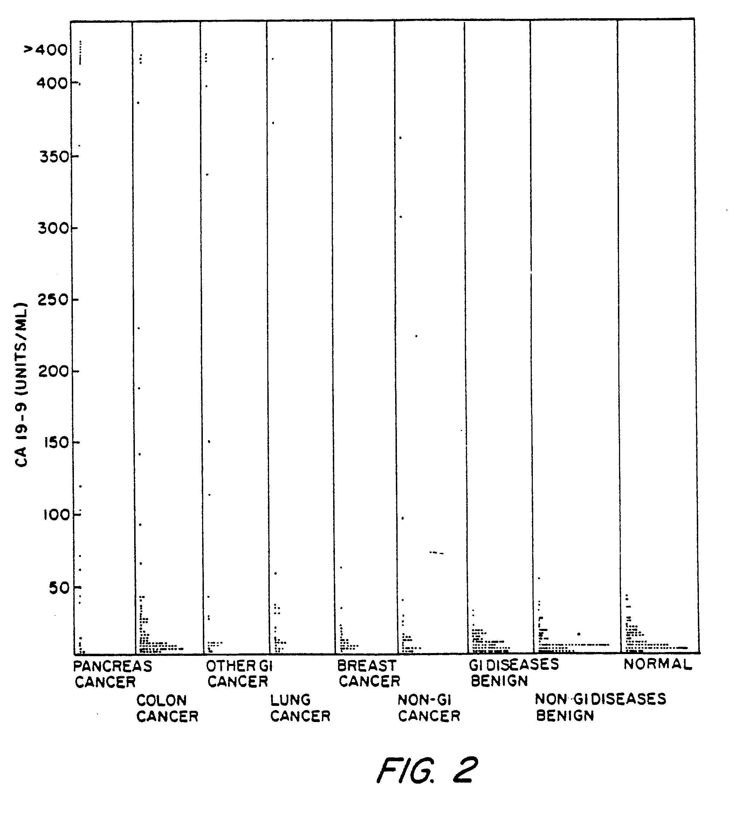

The concentration of CA 19—9 in sera from a coded panel from the Mayo Clinic—NCI and in sera from blood bank donors was determined using the CA 19—9 assay. Four hundred and eleven samples from the Mayo Clinic-NCI Serum Bank were assayed. As shown in Figure 2, the level of CA 19—9 in the serum of cancer patients was frequently higher than that in the serum of normal individuals or of patients with benign diseases. Using an arbitrary value of 37 units/ml to discriminate between positive and negative samples, sera from 19 of 25 (74%) pancreatic cancer patients were found to contain elevated levels of CA 19—9. Among 75 patients with colorectal cancer, 12 (16%) had CA 19—9 levels above 37 units/ml, including 6 of 23 (26%) having distant metastasis, 4 of 25 (16%) Dukes B and C, and 2 of 27 (7%) Dukes A Disease. Elevated levels of CA 19—9 were also observed in 10 of 31 (32%) sera of patients with other gastrointestinal malignancies (Table 1). High concentrations were usually associated with adenocarcinomas of the hepatobiliary system (gall bladder, ampulla of vater, bile ducts); a gastric adenocarcinoma and a hepatoma were also above 37 units/ml. Additionally, 4 of 25 (16%) sera from lung cancer patients, 1 of 25 (4%) sera from breast cancer patients and 3 of 24 (12%) sera from other cancer patients (Table I) had elevated CA 19—9 levels. In two of these cases (357; 94 units/ml) the site of the primary tumor was unknown and in the

# 0 114 818

third (39 units/ml) the site of the primary was the thyroid. Among 131 patients with benign diseases, only one (0.8%), a 47 year-old male with interstitial pneumonitis of unknown etiology had elevated CA 19—9 (50.7 units/ml). As shown in Table 2, the benign disease group represented a broad range of gastrointestinal and non-gastrointestinal diseases, including 11 patients with benign pancreatic diseases, 52 patients with other gastrointestinal diseases, and 68 with non-gastrointestinal diseases. Only 1 of 75 (1.3%) normal persons had elevated CA 19—9 (40.7 units/ml).

The mean CA 19—9 levels obtained for patient sera from all groups in the panel are presented in Table 3. The highest CA 19—9 levels were detected in sera of patients with pancreatic carcinoma (266±266 units/ml). CA 19—9 levels averaged 8.4±7.6 units/ml in normal individuals, 6.5±6.2 units/ml in patients with benign gastrointestinal disorders, 7.1±5.4 units/ml with benign pancreatic disease, and 8.6±9.5 units/ml with benign non-enteric disease. No statistically significant differences in these values could be found when patients were grouped according to age or fasting or nonfeeding, i.e. the CA 19—9 levels appeared to be independent of these parameters in the patient groups studied (data not shown).

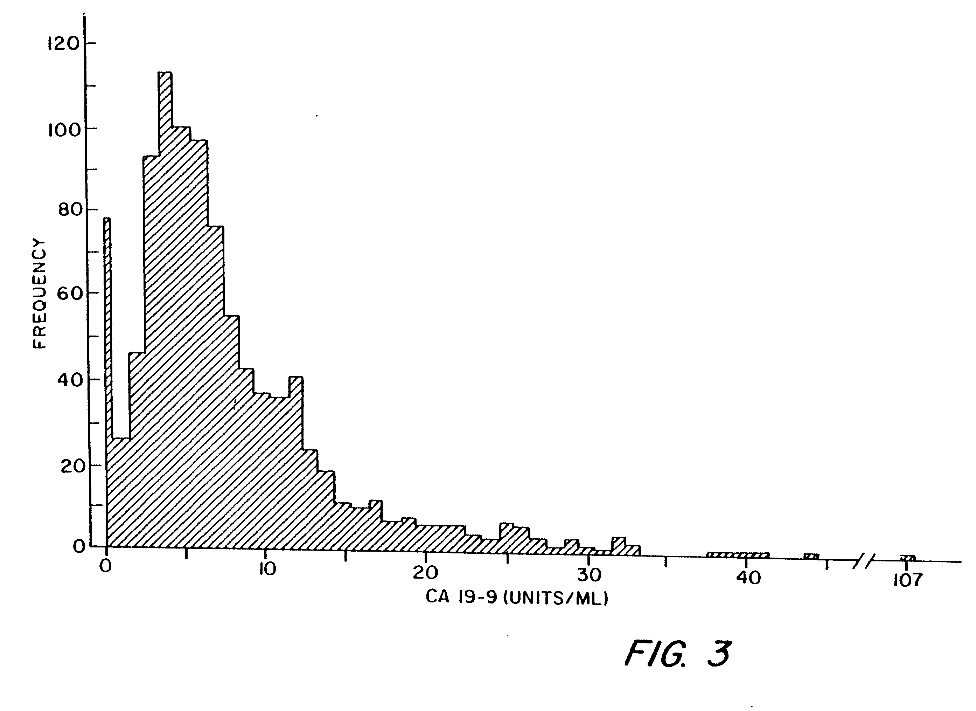

Sera obtained from randomly selected, healthy blood bank donors were evaluated using the CA 19—9 assay. As shown in Figure 3, the mean for the normals was 8.7±7.4 units/ml (range 0—107 units/ml). Only 0.5% of these samples contained CA 19—9 at levels in excess of 37 units/ml. In addition, there was no statistically significant relationship between the age of the donors and the level of CA 19—9 in their sera. Small but statistically significant differences were observed between males and females. Females between 20 and 49 years of age had slightly higher levels than males. No further information could be obtained concerning the donors whose sera had elevated CA 19—9 levels.

The sensitivity of the CA 19—9 RIA for pancreatic cancer (74%) and the specificity among normal individuals (99.5%), and among patients with a wide variety of benign diseases (99.2%) were excellent. These benign diseases included benign pancreatic diseases, diabetes, arthritis and inflammatory gastrointestinal disorders.

CA 19—9 levels above 37 units/ml were observed in several patients with other gastrointestinal malignancies, particularly of the hepatobiliary system. Because the CA 19—9 antigen is present in normal tissues, especially the epithelial cells lining the ductules in the liver and pancreas, more extensive studies among patients with hepatobiliary tract disorders and pancreatitis will be required to assess reactivity in these patients.

CA 19—9 levels above 37 units/ml were observed in relatively few patients with colorectal cancer. Only 7% of patients with Dukes A, 16% of patients with Dukes B or C and 26% of patients with advanced metastatic colorectal disease had elevated CA 19—9 values.

Sera from some patients with non-gastrointestinal malignancies, especially lung tumors, had elevated levels of CA 19—9. In the four positive lung cancer patients, there was no apparent relationship to tumor type, grade or stage: Three were adenocarcinomas, grades 3 and 4, stages I, II, and III. The fourth was a squamous cell carcinoma, grade 3, stage I. Only one breast adenocarcinoma and one thyroid papillary carcinoma had elevated CA 19—9 levels.

To determine the effect of pH, a series of forward sandwich assays, as described above, were performed on samples prepared from one serum sample (PC 2). CA 19—9 antigen was purified from human ascites and then diluted into normal human serum. Samples were prepared by mixing serum-diluted CA 19—9 with either citrate or phosphate buffer and sufficient hydrochloric acid to produce the desired pH. The results are presented in Table 4.

4

**0 114 818**

TABLE 1

CA 19—9 Levels in sera from patients with miscellaneous cancer

| Diagnosis | CA 19—9 (units/ml) |
|---|---|
| Ampulla of vater | 395.7 |
| | 41.6 |
| | 4.2 |
| Stomach | 535.6 |
| | 7.9 |
| | 0.0 |
| Hepatoma | 333.5 |
| | 10.0 |
| Hepatic and cystic duct cancer | 63.2 |
| Cancer bile duct | 151.5 |
| | 112.8 |
| | 23.3 |
| Gall bladder | 640.8 |
| | 528.2 |
| | 5.0 |
| Appendix, carcinoid | 0.0 |
| Ileum | 9.6 |
| | 6.1 |
| | 2.7 |
| Duodenum cancer | 27.3 |
| | 8.2 |
| | 0.1 |
| Esophagus | 45.1 |
| | 7.7 |
| | 6.9 |
| | 6.9 |
| | 6.2 |
| | 5.4 |
| | 4.3 |
| | 3.3 |
| Barrett's esophagus | 5.4 |
| Primary unknown | 357.6 |
| | 93.6 |
| | 10.3 |
| | 0.0 |
| Burkitt's lymphoma | 17.9 |
| Lymphoma (remission) | 20.8 |
| Leukemia | 8.6 |
| | 7.1 |
| Hodgkin's disease | 8.2 |
| Malignant histiocytoma | 3.2 |
| Plasmacytoma (pulmonary) | 0.0 |
| Kidney | 16.5 |
| | 5.9 |
| Bladder | 11.2 |
| Testicular | 9.0 |
| Prostate | 3.7 |
| Thyroid | 39.0 |
| | 0.0 |
| Malignant melanoma | 8.4 |
| Cancer nasopharynx | 0.0 |
| Synovial cell sarcoma | 2.6 |
| Cancer retroperitoneum | 3.3 |
| Osteogenic sarcoma | 2.5 |
| Liposarcoma (thigh) | 1.2 |

## TABLE 2
### CA 19—9 Levels in sera from patients with benign diseases

| Diagnosis | CA 19—9 (units/ml) |
|---|---|
| Arthritis, degenerative | 21.2 |
| | 9.5 |
| | 5.0 |
| | 0.0 |
| Arthritis, osteo | 15.1 |
| | 5.3 |
| Arthritis, rheumatoid | 5.7 |
| | 3.9 |
| | 0.2 |
| Arthritis | 5.4 |
| | 4.7 |
| | 4.4 |
| Degenerative joint disease | 16.0 |
| | 9.1 |
| | 4.4 |
| | 1.9 |
| Hypothyroidism | 4.2 |
| Thyroiditis | 15.9 |
| Thyroiditis, hashimoto's | 15.1 |
| | 5.4 |
| Graves' disease | 14.9 |
| | 5.6 |
| Fibrocystic disease (breast) | 13.7 |
| | 5.0 |
| Gynecomastia | 5.4 |
| | 4.2 |
| Psoriasis pancreatitis | 9.4 |
| Fatty liver | 28.3 |
| Cirrhosis | 25.1 |
| | 13.7 |
| | 7.2 |
| | 6.7 |
| | 4.5 |
| | 2.4 |
| Chronic ulcerative colitis | 15.7 |
| | 3.8 |
| | 3.6 |
| | 1.5 |
| | 0.0 |
| | 0.0 |
| Diarrhoea, unknown etiology | 14.6 |
| | 13.2 |
| Irritable colon/bowel | 6.9 |
| | 6.3 |
| | 4.8 |
| | 4.1 |
| Crohn's disease | 6.8 |
| | 0.0 |
| Achalasia | 5.0 |
| | 0.2 |
| Post vagotomy diarrhoea | 3.0 |
| Pyloric stenosis | 2.6 |
| Pneumonitis (interstitial, etiology unknown) | 50.7 |
| Systemic mast cell disease (C.O.P.D.) | 35.3 |
| Pemphigus—vulgaris | 32.1 |
| Low blood pressure | 29.2 |

TABLE 2 (continued)

| Diagnosis | CA 19—9 (units/ml) |
|---|---|
| Diabetes | 22.5 |
| | 22.4 |
| | 13.4 |
| | 5.0 |
| | 4.3 |
| | 0.0 |
| Pericardial cyst | 19.1 |
| Cholelithiasis | 14.5 |
| Hepatitis, chronic, active | 13.4 |
| | 10.2 |
| | 8.3 |
| | 0.0 |
| Malabsorption syndrome | 13.2 |
| Polyp | 12.3 |
| Ulcer(s) | 11.3 |
| | 8.3 |
| | 3.2 |
| | 3.0 |
| | 2.7 |
| | 2.1 |
| | 1.7 |
| | 0.0 |
| | 0.0 |
| Gastritis, duodenitis | 10.3 |
| | 8.9 |
| | 6.3 |
| | 5.0 |
| | 2.0 |
| Intermediate gastric | 7.5 |
| Motility disorder | 3.9 |
| Gastric hypertension, abdominal pain | 7.3 |
| Granuloma | 11.0 |
| | 10.2 |
| | 4.4 |
| | 4.3 |
| | 2.9 |
| Benign bone cyst | 8.3 |
| Pregnancy | 6.8 |
| Thrombocytopenic purpura | 2.3 |
| Chronic alcoholism | 1.3 |
| Fibrosis | 4.4 |
| Turner's variant | 0.0 |
| Dyspepsia | 0.0 |
| Unknown abdominal pain | 0.0 |
| Alcohol abuse | 4.4 |
| | 4.1 |
| Menometrorrhagia | 3.6 |
| Sarciodosis | 3.1 |
| | 3.0 |
| | 0.0 |
| Hyperplastic mucosa | 3.5 |
| Polyps, cervical | 2.0 |
| Scoliosis | 2.7 |
| Hematoma | 4.9 |
| Thornwaldt's disease | 4.1 |
| Aneurysm | 3.1 |
| Dumping syndrome | 0.0 |
| Benign prostatic hypertrophy | 4.1 |
| Friction blisters | 2.7 |
| Mixed connective tissue disease | 1.8 |

**0 114 818**

TABLE 3

Average CA 19—9 concentration in patient sera

| | Group | n | Mean±S.D. | Percent positive[a] |
|---|---|---|---|---|
| A. | Cancers Pancreas | 25 | 266±266 | 74 |
| | Colon Metastatic | 23 | 81.9±168 | 26 |
| | Dukes B & C | 25 | 59.8±123 | 16 |
| | Localized | 27 | 10.5±14.2 | 7 |
| | GI (misc.) | 31 | 97.3±182 | 32 |
| | Lung | 25 | 52.8±109 | 16 |
| | Breast | 25 | 10.3±12 | 4 |
| | Other | 24 | 26.7±73.1 | 12 |
| B. | Benign diseases Pancreatic | 11 | 7.1±5.4 | 0 |
| | Other GI | 52 | 6.5±6.2 | 0 |
| | Non-GI | 68 | 8.6±9.5 | 1.4 |
| C. | Normals Normals | 75 | 8.4±7.6 | 1.3 |

[a]Positive 37 units/ml

TABLE 4

Effect of buffer pH on CA 19—9 detected

| Buffer pH | CPM $^{125}$I-19—9 |
|---|---|
| 1.5 | 4680 |
| 2.0 | 5970 |
| 2.5 | 8880 |
| 3.0 | 9480 |
| 3.5 | 10860 |
| 4.0 | 23500 |
| 4.5 | 31100 |
| 5.0 | 26400 |
| 5.5 | 22800 |
| 6.0 | 14800 |
| 6.5 | 8260 |
| 7.0 | 4580 |
| 7.5 | 2870 |
| 8.0 | 2180 |

Industrial applicability

The invention described herein is useful in the detection of carbohydrate antigenic determinants, such as CA 19—9. The assay can be employed by hospitals or clinical laboratories for the early detection of tumors, particularly pancreatic cancer tumors.

**Claims**

1. An immunoassay for the carbohydrate antigenic determinant CA 19—9, characterised in that the reaction between CA 19—9 antigen and CA 19—9 antibody is carried out at an acidic pH.

8

**0 114 818**

2. An immunoassay according to claim 1, wherein the pH is from 2.5 to 6.

3. An immunoassay according to claim 1, wherein the pH is about 4.5.

4. An immunoassay according to any one of claims 1, 2 and 3, wherein the immunoassay is a forward sandwich type immunometric assay comprising the step of incubating a liquid sample with a solid phase immunoadsorbent containing CA 19—9 antibody for a period sufficient to allow any CA 19—9 antigen in the sample to bind to the solid phase antibody; separating the immunoadsorbent from the sample; incubating the immunoadsorbent with a labelled antibody capable of binding CA 19—9 antigen; separating the immunoadsorbent from unbound labelled antibody; and detecting the labelled antibody remaining bound to the immunoadsorbent to obtain a measure of the amount of CA 19—9 in the sample; and wherein both antibody-antigen reactions are carried out at the said acidic pH.

**Patentansprüche**

1. Immunotest für die Kohlehydrat-Antigen-Determinante CA 19—9, dadurch gekennzeichnet, daß die Reaktion zwischen dem Antigen CA 19—9 und dem Antikörper CA 19—9 bei einem sauren pH-Wert durchgeführt wird.

2. Immunotest nach Anspruch 1, dadurch gekennzeichnet, daß der pH-Wert 2,5 bis 6 beträgt.

3. Immunotest nach Anspruch 1, dadurch gekennzeichnet, daß der pH-Wert etwa 4,5 beträgt.

4. Immunotest nach einem der Ansprüche 1, 2 und 3, dadurch gekennzeichnet, daß der Immunotest ein immunometrischer Test des fortschreitenden Sandwich-Typs mit den Schritten ist, daß eine Flüssigprobe mit einem den Antikörper CA 19—9 enthaltenden Festphasen-Immunoadsorbens für einen solchen Zeitraum einem Inkubationsprozeß unterzogen wird, daß ein in der Probe enthaltenes Antigen CA 19—9 eine Verbindung mit dem Festphasen-Antikörper eingehen kann, das Immunoadsorbens von der Probe getrennt wird, das Immunoadsorbens mit einem markierten Antikörper, der in der Lage ist, das Antigen CA 19—9 zu binden, einem Inkubationsprozeß unterzogen wird, das Immunoadsorbens von ungebundenen markierten Antikörpern getrennt wird und die an das Immunoadsorbens gebunden verbleibenden markierten Antikörper zum Erhalt eines Maßes für die Menge von CA 19—9 in der Probe festgestellt werden, wobei beide Antikörper-Antigen-Reaktionen bei dem sauren pH-Wert durchgeführt werden.

**Revendications**

1. Immunotest concernant le déterminant antigénique glucidique CA 19—9, caractérisé en ce que la réaction entre l'antigène CA 19—9 et l'anticorps CA 19—9 est conduite à un pH acide.

2. Immunotest selon la revendication 1, dans lequel le pH est compris entre 2,5 et 6.

3. Immunotest selon la revendication 1, dans lequel le pH est d'environ 4,5.

4. Immunotest selon l'une quelconque des revendications 1 à 3, caractérisé en ce que l'immunotest est un test immunométrique du type en sandwich progressif comprenant les phases consistant à mettre un échantillon liquide en incubation avec un immunoadsorbant en phase solide contenant l'anticorps CA 19—9 pendant une periode suffisante pour permettre à un antigène CA 19—9 éventuellement présent dans l'échantillon de se fixer à l'anticorps en phase solide; à séparer l'immunoadsorbant de l'échantillon; à mettre l'immunoadsorbant en incubation avec un anticorps marqué capable de fixer l'antigène CA 19—9; à séparer l'immunoadsorbant de l'anticorps marque non fixé; et a détecter l'anticorps marqué qui reste fixé à l'immunoadsorbant pour obtenir une mesure de la quantité de CA 19—9 dans l'échantillon; et en ce que les deux réactions anticorps-antigène sont conduites audit pH acide.

9

FIG. 1

FIG. 2

FIG. 3